# EUROPEAN PATENT SPECIFICATION

(11) **EP 2 233 137 B1**
(45) Date of publication and mention of the grant of the patent: **30.04.2014**
(21) Application number: 10002380.3
(22) Date of filing: 08.11.2006
(51) Int. Cl.: A61K 31/198, A61K 38/05, A61K 38/06, C07K 5/06, C07K 5/08, G01N 33/566

(54) **Calcium receptor activator**
Kalziumrezeptor-Aktivator
Activateur de récepteur de calcium

(30) Priority: 09.11.2005 JP 2005325301; 22.11.2005 US 738560 P
(43) Date of publication of application: 29.09.2010
(62) Divisional of application: 06823386.5
(73) Proprietor: Ajinomoto Co., Inc., Tokyo 104-8315 (JP)
(72) Inventor: Ohsu, Takeaki, Kawasaki-shi, Kanagawa, 210-8681 (JP); Takeshita, Sen, Kawasaki-shi, Kanagawa, 210-8681 (JP); Eto, Yuzuru, Kawasaki-shi, Kanagawa, 210-8681 (JP); Amino, Yusuke, Kawasaki-shi, Kanagawa, 210-8681 (JP)
(74) Representative: Strehl Schübel-Hopf & Partner

(56) References cited:
- WO-A1-2007/055393
- Anonymous: "Calcium sensing receptor" Wikipedia (English language) 27 August 2010 (2010-08-27), XP002598415 Retrieved from the Internet: URL:http://en.wikipedia.org/wiki/Calcium-s ensing_receptor [retrieved on 2010-08-27]

### SEQUENCE LISTING

<110> Ajinomoto Co., Inc.
<120> Calcium receptor activator
<130> EPA-64872
<150> JP2005-325301
   <151> 2005-11-9
<150> US 60/738560
   <151> 2005-11-22
<160> 4
<170> PatentIn version 3.1
<210> 1
   <211> 49
   <212> DNA
   <213> Artificial
<220>
   <223> hCASR_N primer
<400> 1
   actaatacga ctcactatag ggaccatggc attttatagc tgctgctgg 49
<210> 2
   <211> 29
   <212> DNA
   <213> Artificial
<220>
   <223> hCASR_C primer
<400> 2
   ttatgaattc actacgtttt ctgtaacag 29
<210> 3
   <211> 23
   <212> DNA
   <213> Artificial
<220>
   <223> rCASR_f primer
<400> 3
   atggaaagct cagatgaaat gtc 23
<210> 4
   <211> 24
   <212> DNA
   <213> Artificial
<220>
   <223> rCASR_r primer
<400> 4
   ggagtgtaat acgttttccg tcac 24

## Description

### Technical Field

This application claims priority from Japanese Patent Application No. 2005-325300 filed on November 9, 2005 , US Provisional Patent Application No. 60/738562 filed on November 22, 2005.

The present invention relates to a composition for therapeutic use comprising a calcium receptor activator containing D-Cys or a peptide.

### Background Art

The calcium receptor is also the called calcium sensing receptor (CaSR), and is a receptor consisting of 1078 amino acids, and classified into the class C of seven-transmembrane receptors (G protein-coupled receptor; GPCR). Cloning of the gene for this calcium receptor was reported in 1993 (Non-patent document 1), and it is known to cause various cell responses through elevation of intracellular calcium level etc., when it is activated with calcium etc. The gene sequence of the human calcium receptor is registered with GenBank Accession No. NM_000388, and is well conserved in animals.

The aforementioned calcium receptor may act to promote or suppress biological functions. Therefore, at present, therapeutic agents which may act as activators and inhibitors of the calcium receptor are appropriately used in the treatment of neurological diseases, hepatic diseases, cardiovascular diseases, digestive system diseases, and other diseases, depending on pathological conditions. For example, the calcium receptor is able to detect increased blood calcium level in the parathyroid, and suppress secretion of the parathyroid hormone (PTH) to correct the blood calcium level. Therefore, an effect of reducing the blood calcium level is expected for a calcium receptor activator. It has been actually clarified that when a calcium receptor activator is used to treat secondary hyperparathyroidism in a hemodialysis patient, it reduces the PTH level without elevating the calcium and phosphorus levels.

Since functional analysis of the calcium receptor has been conducted mainly for calcium homeostasis, the applied researches therefor are so far mainly concerned bone metabolic diseases in which calcium regulation is involved. However, it has become clear that the calcium receptor is widely distributed in living bodies other than the parathyroid and kidney from the results of genetic expression analyses etc. (Non-patent document 2, Non-patent document 3), and possibilities thereof for being involved in various biological functions and causes of diseases have been proposed. For example, it is estimated that the calcium receptor is involved in the functions of the liver, heart, lung, alimentary canal, lymphocyte, and pancreas. The inventors of the present invention have also confirmed that it is expressed in a wide range of tissues in living bodies by analyses based on RT-PCR using RNAs extracted from rat tissues. From the aforementioned viewpoints, the values of activators and inhibitors of the calcium receptor are presently rapidly increasing for applications.

Moreover, other than calcium, cations such as gadolinium cation, basic peptides such as polyarginine, polyamine such as spermine, amino acids such as phenylalanine, and so forth have been reported, as calcium receptor activators (Non-patent document 4).

Although many specific activators have been developed so far as calcium receptor activators as described above, there are few compounds existing in living bodies among them, and the activities of the compounds existing in living bodies are very low. Therefore, therapeutic agents for various diseases containing these activators have serious problems concerning side reaction, permeability and activity. For example, although it is known that amino acids act on the calcium receptor, it is considered that actual application thereof as the activators is difficult, since the activity is very weak. Moreover, although macromolecules such as polyarginine have been reported as the activator as described above, it is estimated that the functions are based on the actions as polyvalent cations having irregular structures. That is, it is not known that a peptide having a specific structure is useful as a calcium receptor activator.
[Non-patent document 1] Nature, 1993 Dec. 9, 366(6455):575-80
[Non-patent document 2] J. Endocrinol., 2000 May, 165(2):173-7
[Non-patent document 3] Eur. J. Pharmacol., 2002 Jul. 5, 447(2-3):271-8
[Non-patent document 4] Cell Calcium, 2004 Mar., 35(3) :209-16

### Disclosure of Invention

### [Problems to be solved by the Invention]

An object of the present invention is to provide a highly active calcium receptor activator highly safe to living bodies for use in therapeutically reducing blood calcium level or parathyroid hormone level.

### [Means for solving the Problems]

The inventors of the present invention searched for activators of the calcium receptor, and as a result, found that low molecular peptides, including glutathione, were able to activate the calcium receptor. The present invention was accomplished on the basis of this finding. That is, the present invention provides the following.
[1] A composition comprising one or more kinds of substances selected from γ-Glu-Cys, γ-Glu-Cys(SNO)-Gly, γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-Glu-Val-Lys, γ-Glu-y-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys(S-Me), γ-Glu-Abu-Gly and γ-Glu-Cys(S-Me)-Gly for use in therapeutically reducing blood calcium level or parathyroid hormone level.
[2] The composition for use according to [1] above, which further contains an other compound having calcium receptor activation activity which is a cation.

### Effect of the Invention

By the present invention, a therapeutical use of a highly active calcium receptor activator highly safe to living bodies is provided.

### Brief Description of the Drawings

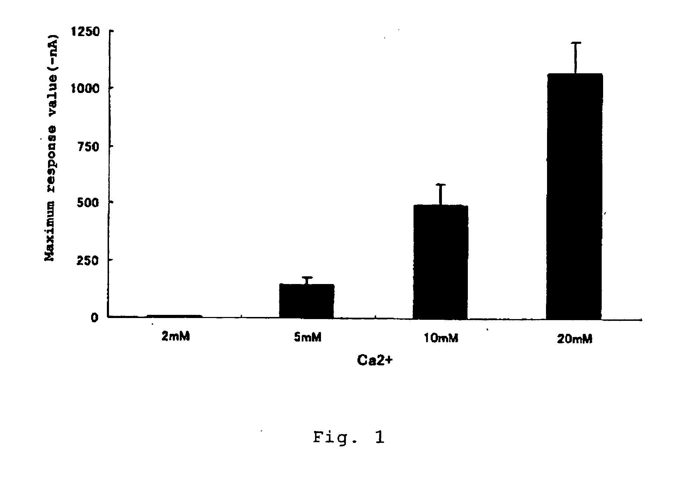

[Fig. 1] Drawing showing an action of calcium on the calcium receptor.
   The human calcium receptor cRNA was introduced into *Xenopus laevis* oocytes by microinjection. Recorded were values of intracellular response currents which flew when a calcium chloride solution was added at an arbitrary concentration. The maximum values of intracellular currents were considered response current values. It was confirmed that no response was observed in oocytes introduced with distilled water by microinjection as a control.

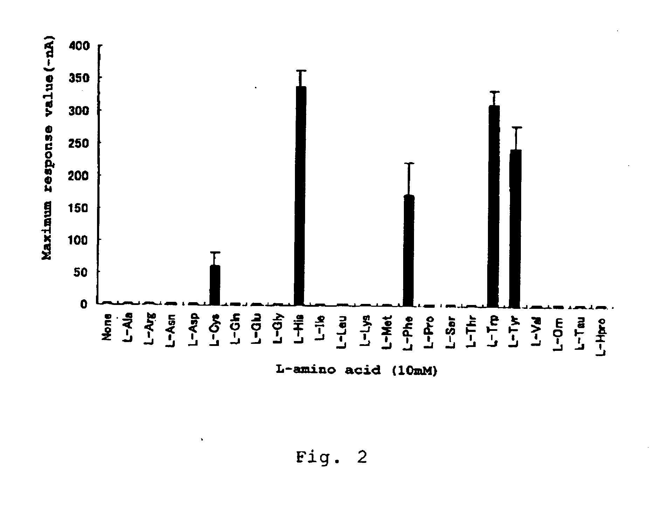

[Fig. 2] Drawing showing an action of L-amino acids on the calcium receptor.
   The human calcium receptor cRNA was introduced into *Xenopus laevis* oocytes by microinjection. Recorded were values of intracellular response currents which flew when a 10 mM L-amino acid solution was added. The maximum values of intracellular currents were considered response current values. It was confirmed that no response was observed in oocytes introduced with distilled water by microinjection as a control.

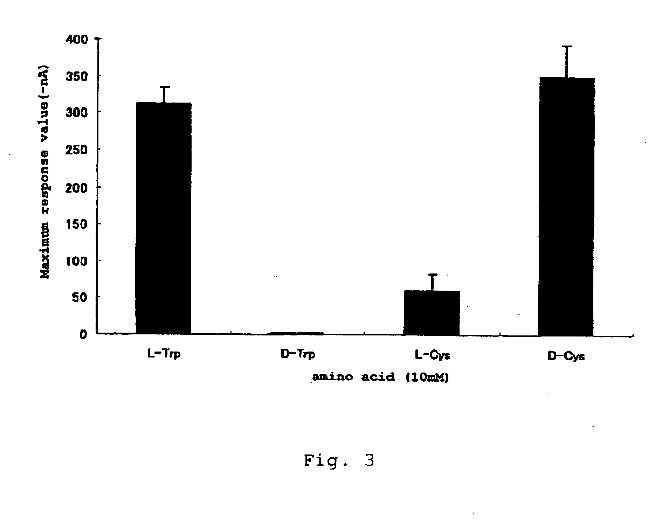

[Fig. 3] Drawing showing an action of D-amino acids on the calcium receptor.
   The human calcium receptor cRNA was introduced into *Xenopus laevis* oocytes by microinjection. Recorded were values of intracellular response currents which flew when a 10 mM D-amino acid solution was added. The maximum values of intracellular currents were considered response current values. It was confirmed that no response was observed in oocytes introduced with distilled water by microinjection as a control.

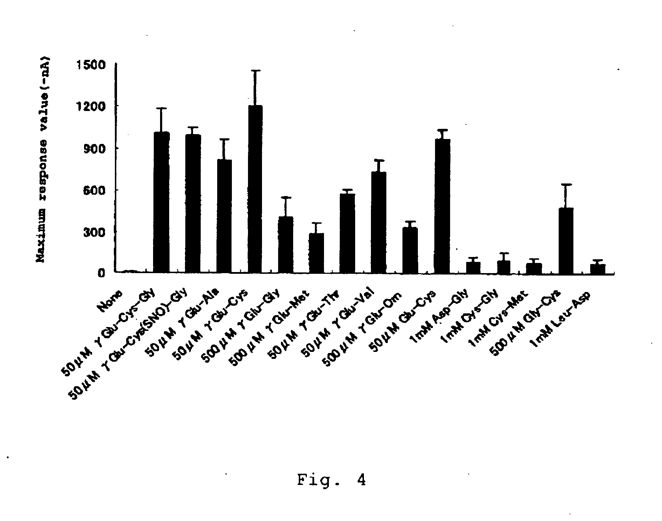

[Fig. 4] Drawing showing an action of peptides on the calcium receptor.
   The human calcium receptor cRNA was introduced into *Xenopus laevis* oocytes by microinjection. Recorded were values of intracellular response currents which flew when a peptide solution was added at an arbitrary concentration. The maximum values of intracellular currents were considered response current values. It was confirmed that no response was observed in oocytes introduced with distilled water by microinjection as a control.

### Best Mode for Carrying out the Invention

Hereafter, the present invention will be explained in detail.

The calcium receptor activator used in the present invention contains one or more kinds of substances selected from γ-Glu-Cys(SNO)-Gly, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-Glu-Val-Lys, γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys(S-Me), γ-Glu-Abu-Gly, γ-Glu-Cys(S-Me)-Gly, and γ-Glu-Val-Gly. In this specification, all the amino acids and amino acid residues constituting the peptides are L-isomers, unless otherwise specified.

In the present specification, abbreviations for amino acid residues are as follows:
(1) Gly: Glycine
(2) Ala: Alanine
(3) Val: Valine
(4) Leu: Leucine
(5) Ile: Isoleucine
(6) Met: Methionine
(7) Phe: Phenylalanine
(8) Tyr: Tyrosine
(9) Trp: Trptophan
(10) His: Histidine
(11) Lys: Lysine
(12) Arg: Arginine
(13) Ser: Serine
(14) Thr: Threonine
(15) Asp: Aspartic acid
(16) Glu: Glutamic acid
(17) Asn: Aspargine
(18) Gln: Glutamine
(19) Cys: Cysteine
(20) Pro: Proline
(21) Orn: Ornithine
(22) Sar: Sarcosine
(23) Cit: Citruline
(24) N-Val: Norvaline
(25) N-Leu: Norleucine
(26) Abu: alpha-Aminobutylic acid
(27) Tau: Taurine
(28) Hyp: Hydroxyproline
(29) t-Leu: tert-Leucine
(30) Cys (S-Me): S-methyl cysteine
(31) Cys (S-allyl): S-allyl cysteine
(32) Val-NH₂: valinamide
(33) Val-ol: valinol (2-amino-3-methyl-l-butanol)

The aforementioned γ-Glu-Cys(SNO)-Gly has the following structural formula, and the "(O)" in the formulas γ-Glu-Met(O) and γ-Glu-Cys(S-Me)(O) indicates a sulfoxide structure. The "(γ)" in the γ-Glu indicates that glutamic acid bonds to another amino acid via γ position of carboxy group in the glutamic acid.

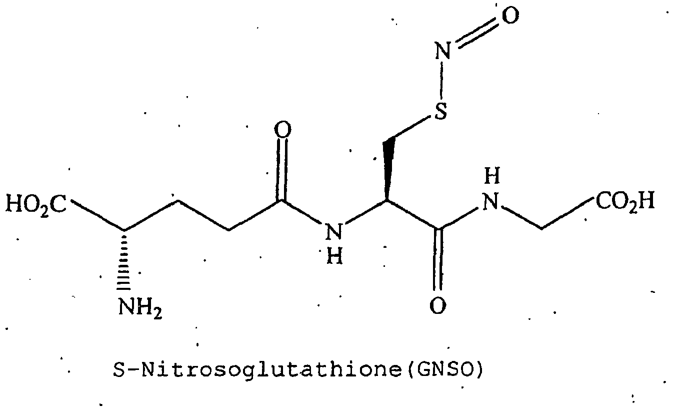

In this specification, the "calcium receptor" is also the called calcium sensing receptor (CaSR), and belongs to the class C of seven-transmembrane receptors. In this specification, the "calcium receptor activator" is a substance that acts on the aforementioned calcium receptor to activate the calcium receptor and control the functions of cells which express the calcium receptor. Furthermore, in this specification, "to activate the calcium receptor" indicates when a ligand (activator) binds to the aforementioned calcium receptor, resulting in activation of the guanine nucleotide binding protein, which thereby transmits signals.

It was demonstrated by the inventors of the present invention that γ-Glu-Cys-(SNO)-Gly, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-Glu-Val-Lys, γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-ser-Gly, γ-Glu-Cys(S-Me), γ-Glu-Abu-Gly, γ-Glu-Cys(S-Me)-Gly, and γ-Glu-Val-Gly activated the calcium receptor. Therefore, γ-Glu-Cys(SNO)-Gly, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-glut Val-Lys, γ-Glu-γ-Glu-Val, γ-Glu-val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-allyl)-Gly; γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys(S-Me), γ-Glu-Abu-Gly, γ-Glu-Cys(S-. Me)-Gly, and γ-Glu-Val-Gly (henceforth also referred to as the "peptides and amino acids used for the present invention") can be used as activators of the calcium receptor. In the present invention, the peptides and amino acids used for the present invention may be independently used, or can be used as a mixture of arbitrary two or more kinds of them.

As the aforementioned peptides and amino acids, if they are available as commercial products, those can be used. Furthermore, the peptides can be obtained by suitably using a known technique such as (1) a method of chemically synthesizing them, or (2) a method of synthesizing them by an enzymatic reaction. Since the number of residues of the contained amino acid residues of the peptides used for the present invention is as comparatively small as 2 or 3 residues, a method of chemically synthesizing them is convenient. When chemically synthesizing them, the oligopeptides can be synthesized or half-synthesized by using a peptide synthesizer. Examples of the method of chemically synthesizing them include, for example, a peptide solid phase synthetic method. Peptides synthesized as described above can be purified by usual means, for example, ion exchange chromatography, reversed phase high performance liquid chromatography, affinity chromatography, and so forth. Such a peptide solid phase synthetic method and the following purification of peptide are well known in this technical field.

Furthermore, the peptides used for the present invention can also be prepared by an enzymatic reaction. For example, the method described in International Patent Publication WO2004/01165 can be used. That is, they can also be prepared by reacting one amino acid or dipeptide of which carboxyl terminus is esterified or amidated and an amino acid having a free amino group (for example, an amino acid of which carboxyl group is protected) in the presence of a peptide producing enzyme, and purifying the produced dipeptide or tripeptide. Examples of the peptide producing enzyme include a culture of microorganisms having an ability to produce peptides, microbial cells separated from such culture, processed product of cells of such microorganisms, peptide producing enzymes derived from such microorganisms, and so forth.

The peptides and amino acids used for the present invention also include those in the form of a salt. When the peptides and amino acids used for the present invention are in the form of a salt, they may be pharmacologically acceptable salts. Examples of a salt with an acidic group such as a carboxyl group in the formula include ammonium salts, salts with alkali metals such as sodium, and potassium, salt with alkaline earth metals such as calcium, and magnesium, aluminum salts, zinc salts, salts with organic amines such as triethylamine, ethanolamine, morpholine, pyrrolidine, piperidine, piperazine, and dicyclohexylamine, and salts with basic amino acids such as arginine, and lysine. Examples of salts with a basic group in case that a basic group exists in the formula include salts with inorganic acids such as hydrochloric acid, sulfuric acid, phosphoric acid, nitric acid, and hydrobromic acid, salts with organic carboxylic acids such as acetic acid, citric acid, benzoic acid, maleic acid, fumaric acid, tartaric acid, succinic acid, tannic acid, butyric acid, hibenzoic acid, pamoic acid, enanthoic acid, decanoic acid, teoclic acid, salicylic acid, lactic acid, oxalic acid, mandelic acid, and malic acid, and salts with organic sulfonic acids such as methanesulfonic acid, benzenesulfonic acid, and p-toluenesulfonic acid.

As the aforementioned calcium receptor, the human calcium receptor encoded by the human calcium receptor gene registered with GenBank Accession No. NM_000388 can be exemplified as a preferred example. In addition, the calcium receptor is not limited to the protein encoded by the gene of the aforementioned sequence, and it may be a protein encoded by a gene having a homology of 60% or more, preferably 80% or more, more preferably 90% or more, to the aforementioned sequence, so long as the protein has the calcium receptor function. The GPRC6A receptor and the 5.24 receptor are also known as subtypes of the calcium receptor, and they can be used for the present invention. The calcium receptor function can be examined by expressing a gene of interest in a cell and measuring changes in the electric current, or intracellular calcium ion concentration at the time of the addition of calcium.

The calcium receptor activator used in the present invention may be a substance that activates, besides the aforementioned human calcium receptor, any one of calcium receptors derived from animals such as mouse, rat, and dog.

Whether the peptides and amino acids used for the present invention activate a calcium receptor can be confirmed by using live cells expressing a calcium receptor or a fragment thereof, cell membranes expressing a calcium receptor or a fragment thereof, an in vitro system containing a protein of a calcium receptor or a fragment thereof, or the like.

An example using live cells is shown below.

A calcium receptor is expressed in cultured cells such as those of *Xenopus laevis* oocytes, hamster ovarian cells, and human fetal kidney cells. The calcium receptor can be expressed by cloning a calcium receptor gene in a plasmid that can contain a foreign gene and introducing the plasmid or cRNA obtained by using the plasmid as a template. To detect the reaction, electrophysiological techniques, fluorescent indicator reagents that indicate an increase in intracellular calcium level, and so forth, can be used.

Expression of the calcium receptor is first confirmed based on the response to calcium or a specific activator. Oocytes that showed intracellular current with calcium at a concentration of about 5 mM, or cultured cells that showed fluorescence of the fluorescent indicator reagent with calcium at a concentration of about 5 mM are used. Calcium concentration dependency is determined by changing the calcium concentration. Then, a test substance such as a peptide is prepared to a concentration of about 1 µM to 1 mM, and added to the oocytes or cultured cells, and whether the aforementioned peptide or the like acts as an activator for the calcium receptor is determined.

Furthermore, the calcium receptor activator used in the present invention may further contain one or more kinds of compounds having calcium receptor activation activity, in addition to one or more kinds of substances selected from γ-Glu-Cys-Gly, γ-Glu-Ala, γ-Glu-Gly, γ-Glu-Cys, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-Glu-Val-Lys, γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys(S-Me), γ-Glu-Abu-Gly, γ-Glu-Cys(S-Me)-Gly, and γ-Glu-Val-Gly mentioned above.

Examples of the existing compounds having aforementioned calcium receptor activation activity include cations such as calcium and gadolinium cations, basic peptides such as polyarginine and polylysine, polyamines such as putrescine, spermine and spermidine, proteins such as protamine, amino acids such as phenylalanine, cinacalcet, and so forth, but is not limited to these. In the present invention, the aforementioned existing compounds having calcium receptor activation activity may be added alone, or as a mixture of two or more kinds. Among the aforementioned existing compounds having calcium receptor activation activity, cations such as calcium and gadolinium cations are preferred, and the calcium cation is more preferred. That is, it is preferred that at least one kind of the existing calcium receptor activators further added should be a cation.

By the coexistence of the aforementioned existing compounds having calcium receptor activation activity, stronger activation of the calcium receptor can be obtained. The ratio of the total amount of the peptides and amino acids used for the present invention and the total amount of the existing compounds having calcium receptor activation activity in the calcium receptor activator used in the present invention is not particularly limited, so long as stronger activation of the calcium receptor can be obtained. However, for example, the mass ratio of the total amount of the peptides and amino acids used for the present invention and the total amount of the existing compounds having calcium receptor activation activity is preferably 1:100 to 100:1.

The calcium receptor is expressed in various tissues and is responsible for various physiological functions. Moreover, activators of the calcium receptor have been developed as therapeutic drugs to treat medical diseases (for example, Therapeutic drugs of hypercalcemia, Proc. Natl. Acad. Sci. USA, 1998 Mar. 31, 95(7):4040-5).

Other than the control of calcium, it has been reported that, as for adipocytes, the calcium receptor is expressed in both mature adipocytes and undifferentiated adipocytes, and is involved in suppression of differentiation (Endocrinology, 2005 May, 146(5):2176-9; Exp. Cell Res., 2004 Dec., 10;301(2):280-92), as for bone marrow cells, it is expressed in erythroblasts, megakaryocytes and thrombocytes, and is involved in hematogenous regulation (J. Bone Miner Res., 1997 Dec., 12 (12):1959-70.), and it is expressed in gastric parietal cells and is involved in gastric acid secretion (J. Clin. Endocrinol. Metab., 2005 Mar., 90(3):1489-94). In addition to the above, it has been suggested that it is expressed in the duodenum, jejunum, ileum (Am. J. Physiol. Gastrointest. Liver Physiol., 2002 Jul., 283(1):G240-50.), large intestine (Am. J. Physiol. Gastrointest. Liver Physiol., 2002 Jul., 283(1):G240-50.), epidermal keratinocytes (Cell Calcium, 2004 Mar., 35(3):265-73.), hepatic cells (J. Biol. Chem., 2001 Feb. 9, 276(6):4070-9), vortex lentis (Biochem. Biophys. Res. Commun., 1997 Apr. 28, 233(3):801-5), Langerhans' islets in the pancreas (Endocrine., 1999 Dec., 11(3):293-300.), lung (J. Clin. Endocrinol. Metab., 1998 Feb., 83(2):703-7.), monocyte type cells (J. Clin. Invest., 2000 May, 105(9):1299-305.), osteoblasts (Endocrinology, 2004 Jul., 145(7):3451-62; Am. J. Physiol. Endocrinol. Metab., 2005 Mar., 288(3):E608-16, Epub. 2004 Nov. 16), and so forth, and is involved in functional regulation of these tissues.

Therefore, the calcium receptor activator used in the present invention can be used as an active ingredient in a pharmaceutical composition for preventing or treating a disease in which the calcium receptor is involved. Examples of the disease in which the calcium receptor is involved include medical diseases, surgical diseases, pediatric diseases, orthopedic diseases, plastic surgery diseases, neurosurgical diseases, dermatological diseases, urological diseases, obstetric or gynecological diseases, ophthalmologic diseases, otorhinolaryngological diseases, dentistry or oral surgery diseases, and so forth.

The method for applying a pharmaceutical composition which contains the calcium receptor activator used in the present invention as an active ingredient is not particularly limited, and oral administration, invasive administration utilizing injection or the like, administration of suppositories, or dermal administration can be employed. The active ingredient can be mixed with a non-toxic pharmaceutical carrier in the form of solid or liquid suitable for administration methods such as oral administration and administration by injection, and administered in the form of a conventional pharmaceutical preparation. Examples of such a pharmaceutical preparation include, for example, solid preparations such as tablets, granules, powders, and capsules, liquid preparations such as solutions, suspensions, and emulsions, lyophilized preparations, and so forth. These preparations can be prepared by conventional means for the preparation of drugs.

Examples of the aforementioned non-toxic pharmaceutical carrier include, for example, glucose, lactose, sucrose, starch, mannitol, dextrin, aliphatic acid glycerides, polyethylene glycol, hydroxyethylstarch, ethylene glycol, polyoxyethylene sorbitan fatty acid esters, gelatin, albumin, amino acids, water, physiological saline, and so forth. Moreover, conventional additives such as stabilizers, wetting agents, emulsifiers, binders, and isotonic agents can also be optionally added as required.

The dose of the pharmaceutical composition may be a quantity which is effective for a therapeutic and prophylactic treatment, and can be suitably adjusted depending on age, sex, weight, symptoms, and so forth of patients. However, for oral administration, for example, it is preferably 0.01 to 10 g, more preferably 0.1 to 1 g, in terms of the total amount of the peptides and amino acids used for the present invention, per 1 kg of body weight for a one time administration. Frequency of the administration is not particularly limited, and it can be administered once to several times per day.

The calcium receptor activator used in the present invention can also be used as a food or drink which is effective for treatment or prevention of a disease in which the calcium receptor is involved. For example, it can be prepared as a food or drink indicated that it has a curative or preventive effect for such diseases in which the calcium receptor is involved as mentioned above on a container or package thereof.

The calcium receptor activator used in the present invention can be used to screen for a calcium receptor activation inhibitor. Although a screening method will be exemplified below, the screening method is not limited to this method.

By adding any one of the peptides and amino acids used for the present invention and a test substance to Xenopus oocytes or cultured cells derived from a mammal expressing the calcium receptor, measuring the intracellular electric current or intracellular calcium concentration, and choosing a compound that inhibits increasing the intracellular electric current or calcium concentration caused by the peptide or amino acid used for the present invention, a calcium receptor activation inhibitor can be screened for.

As the test substance used for the screening, low molecular weight compounds, saccharides, peptides, proteins, and so forth can be used.

### Examples

Hereinafter, the present invention will be more specifically explained with reference to examples.

### <Example 1>

### <Preparation of gene (cRNA)>

The gene of the calcium receptor was prepared as follows. On the basis of the DNA sequence registered at NCBI (calcium receptor: NM_000388), synthetic oligo DNAs (forward primer (N) and reverse primer (C)) used for PCR were designed (Table 1, SEQ ID NOS: 1 and 2).

**Table 1**

| Synthetic oligo DNAs (forward primer (N) and reverse primer (C), h: human, r: rat) | |
|---|---|
| Code | Sequence(5'-3') |
| hCASR_N | ACTAATACGACTCACTATAGGGACCATGGCATTTTATAGCTGCTGCTGG |
| hCASR_C | TTATGAATTCACTACGTTTTCTGTAACAG |
| rCASR_f | ATGGAAAGCTCAGATGAAATGTC |
| rCASR_r | GGAGTGTAATACGTTTTCCGTCAC |

By using human kidney cDNA (Clontech) as a material, the primers shown in Table 1 (hCASR_N (SEQ ID NO: 1) and hCASR_C (SEQ ID NO: 2)) were synthesized, and PCR was performed by using Pfu ultra DNA Polymerase (Stratagene) under the following conditions. After a reaction at 94°C for 3 minutes, a cycle of reactions at 94°C for 30 seconds, 55°C for 30 seconds, and 72°C for 2 minutes was repeated 35 times, and then a reaction was performed at 72°C for 7 minutes. Whether amplification was attained by PCR was detected by performing agarose electrophoresis, staining with a DNA staining reagent, and ultraviolet irradiation. The chain lengths of the PCR products were confirmed by comparison with DNA markers of known sizes simultaneously subjected to the electrophoresis. The plasmid vector pBR322 (Takara) was digested with the restriction enzyme EcoRV. The gene fragment amplified by PCR was ligated to the cleavage site of the plasmid by using Ligation Kit (Promega). The *Escherichia coli* DH5α strain was transformed with each ligation reaction solution, and a transformant harboring the plasmid in which the PCR amplification product was cloned was selected. The PCR amplification product was confirmed by DNA sequence analysis. By using this recombinant plasmid as a template together with a cRNA preparation kit (Ambion), cRNA of the calcium receptor gene was prepared.

### <Example 2>

### <Preparation of various samples>

As L-amino acid samples, 23 kinds of special grade amino acids, alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, ornithine, taurine (all of these from Ajinomoto), and hydroxyproline (Nakarai Tesque), were used. As D-Cys and D-Trp (Nakarai Tesque) and calcium chloride, those of special grade were used. Furthermore, as peptide samples, γ-Glu-Cys-Gly (Sigma Aldrich Japan), γ-Glu-Cys(SNO)-Gly (Dojin Chemical Laboratory), γ-Glu-Ala (Bachem Feinchemikalien AG), γ-Glu-Gly (Bachem Feinchemikalien AG), γ-Glu-Cys (Sigma Aldrich Japan), γ-Glu-Met (Bachem Feinchemikalien AG), γ-Glu-Abu-Gly (Abu: α-aminobutyric acid, Bachem Feinchemikalien AG), γ-Glu-Thr (Kokusan Chemical), γ-Glu-Val (Kokusan Chemical), γ-Glu-Leu (contract manufactured product), γ-Glu-Ile (contract manufactured product), γ-Glu-Orn (Kokusan Chemical), Asp-Gly (contract manufactured product), Cys-Gly (contract manufactured product), Cys-Met (contract manufactured product), Glu-Cys (contract manufactured product), Gly-Cys (contract manufactured product), Leu-Asp (contract manufactured product),γ-Glu-Val-Val (contract manufactured product), γ-Glu-Val-Glu (contract manufactured product), γ-Glu-Val-Lys (contract manufactured product), γ-Glu-y-Glu-Val (contract manufactured product), γ-Glu-Gly-Gly (contract manufactured product), γ-Glu-Val-Phe (contract manufactured product), γ-Glu-Val-Ser (contract manufactured product), γ-Glu-Val-Pro (contract manufactured product), γ-Glu-Val-Arg (contract manufactured product), γ-Glu-Val-Asp (contract manufactured product), γ-Glu-Val-Met (contract manufactured product), γ-Glu-Val-Thr (contract manufactured product), γ-Glu-Val-His (contract manufactured product), γ-Glu-Val-Asn (contract manufactured product), γ-Glu-Val-Gln (contract manufactured product), γ-Glu-Val-Cys (contract manufactured product), γ-Glu-Val-Orn (contract manufactured product) and γ-Glu-Ser-Gly (contract manufactured product) were used. Glutamine and cysteine were prepared upon use, and the other samples were stored at -20°C after preparation. As the peptides, those having a purity of 90% or higher were used. As only for γ-Glu-Cys, one having a purity of 80% or higher was used. When the solution dissolving each sample showed an acidic or alkaline pH, the solution was adjusted to an approximately neutral pH by using NaOH or HCl. The solution used for dissolution of amino acids and peptides, preparation of *Xenopus laevis* oocytes, and culture of the oocytes had the following composition: 96 mM NaCl, 2 mM KCl, 1 mM MgCl₂, 1.8 mM CaCl₂, 5 mM Hepes, pH 7.2.

### <Example 3>

### <Synthesis of γ-Glu-Val-Gly>

Boc-Val-OH (8.69 g, 40.0 mmol) and Gly-OBzl•HCl (8.07 g, 40.0 mmol) were dissolved in methylene chloride (100 ml), and the solution was kept at 0°C. Triethylamine (6.13 ml, 44.0 mmol), HOBt (1-hydroxybenzotriazole, 6.74 g, 44.0 mmol), and WSC•HCl (1-ethyl-3-(3-dimethylaminopropyl)carbodiimide hydrochloride, 8.44 g, 44.0 mmol) were added to the solution, and the mixture was stirred overnight at room temperature. The reaction mixture was concentrated under reduced pressure, and the residue was dissolved in ethyl acetate (200 ml). The solution was washed with water (50 ml), 5% citric acid aqueous solution (50 ml x twice), saturated brine (50 ml), 5% sodium hydrogencarbonate aqueous solution (50 ml x twice), and saturated brine (50 ml). The organic layer was dried over anhydrous magnesium sulfate, magnesium sulfate was removed by filtration, and the filtrate was concentrated under reduced pressure. The residue was recrystallized from ethyl acetate/n-hexane to obtain Boc-Val-Gly-OBzl (13.2 g, 36.2 mmol) as white crystals.

Boc-Val-Gly-OBzl (5.47 g, 15.0 mmol) was added to 4 N HCl/dioxane solution (40 ml), and the mixture was stirred at room temperature for 50 minutes. Dioxane was removed by concentration under reduced pressure, n-hexane (30 ml) was added to the residue, and the mixture was concentrated under reduced pressure. This procedure was repeated 3 times to quantitatively obtain H-Val-Gly-OBzl•HCl.

The above H-Val-Gly-OBzl•HCl and Z-Glu-OBzl (5.57 g, 15.0 mmol) were dissolved in methylene chloride (50 ml), and the solution was kept at 0°C. Triethylamine (2.30 ml, 16.5 mmol), HOBt (1-hydroxybenzotriazole, 2.53 g, 16.5 mmol), and WSC•HCl (1-ethyl-3-(3-dimethylaminopropyl)carbodiimide hydrochloride, 3.16 g, 16.5 mmol) were added to the solution, and the mixture was stirred at room temperature for 2 days. The reaction mixture was concentrated under reduced pressure, and the residue was dissolved in heated ethyl acetate (1500 ml). The solution was washed with water (200 ml), 5% citric acid aqueous solution (200 ml x twice), saturated brine (150 ml), 5% sodium hydrogencarbonate aqueous solution (200 ml x twice), and saturated brine (150 ml). The organic layer was dried over anhydrous magnesium sulfate, magnesium sulfate was removed by filtration, and the filtrate was concentrated under reduced pressure. The deposited crystals were collected by filtration, and dried under reduced pressure to obtain Z-Glu(Val-Gly-OBzl)-OBzl (6.51 g, 10.5 mmol) as white crystals.

The above Z-Glu(Val-Gly-OBzl)-OBzl (6.20 g, 10.03 mmol) was suspended in ethanol (200 ml), and added with 10% palladium/carbon (1.50 g), and reduction reaction was performed at 55°C for 5 hours under a hydrogen atmosphere. During the reaction, 100 ml in total of water was gradually added. The catalyst was removed by filtration using a Kiriyama funnel, and the filtrate was concentrated under reduced pressure to a half volume. The reaction mixture was further filtered through a membrane filter, and the filtrate was concentrated under reduced pressure. The residue was dissolved in a small volume of water, and added with ethanol to deposit crystals, and the crystals were collected by filtration, and dried under reduced pressure to obtain white powder of γ-Glu-Val-Gly (2.85 g, 9.40 mmol). ESI-MS:(M+H)⁺ = 304.1
¹H-NMR (400 MHz, D₂O) δ (ppm): 0.87 (3H, d, J=6.8 Hz), 0.88 (3H, d, J=6.8 Hz), 1.99-2.09 (3H, m), 2.38-2.51 (2H, m) 3.72 (1H, t, J=6.35 Hz), 3.86 (1H, d, J=17.8 Hz), 3.80 (1H, d, J=17.8 Hz), 4.07 (1H, d, J=6.8 Hz)

### <Example 4>

### <Synthesis of γ-Glu-Cys(S-Me)-Gly [Cys(S-Me): S-methylcysteine]>

Reduced glutathione (15.0 g, 48.8 mmol) was added to water (45 ml), and sodium hydroxide (4.52 g, 2.2 equivalents, 107 mmol) was added portionwise to the mixture while the mixture was bubbled with nitrogen. Methyl iodide (4.56 ml, 1.5 equivalents, 73 mmol) was added to the mixture, and the mixture was sealed and stirred at room temperature for 2 hours. The reaction mixture was adjusted to pH 2 to 3 with concentrated hydrochloric acid, added with ethanol (150 ml), and stored overnight in a refrigerator. Since oily matter was separated, the supernatant was removed. When the remained oily matter was dissolved in water and gradually added with ethanol, partially crystallized oily matter was deposited. Therefore, the supernatant liquid was removed again. The residue was dissolved in water (300 ml), adsorbed on an ion exchange resin (Dowex 1-acetate, 400 ml) filled in a column, and after washing with water, eluted with 1 N acetic acid aqueous solution. The eluate was concentrated under reduced pressure, and precipitated from water/ethanol to obtain a white powder of γ-Glu-Cys(S-Me)-Gly (5.08 g, 15.8 mmol).
FAB-MS: (M+H)⁺ = 322
¹H-NMR (400 MHz, D₂O) δ (ppm): 2.14 (3H, s), 2.15-2.22 (2H, m), 2.50-2.58 (2H, m), 2.86 (1H, dd, J=9.0 Hz, J=14.0 Hz), 3.03 (1H, dd, J=5.0 Hz, J=14.0 Hz), 3.84 (1H, t, J=6.5 Hz), 3.99 (2H, S), 4.59 (1H, dd, J=5.0 Hz, J=9.0 Hz)

### <Example 5>

### <Synthesis of other peptides>

γ-Glu-Met(O), γ-Glu-Val-NH₂, γ-Glu-Val-ol, γ-Glu-Ser, γ-Glu-Tau, γ-Glu-Cys(S-Me)(O), γ-Glu-t-Leu, γ-Glu-Cys(S-allyl)-Gly, and γ-Glu-Cys(S-Me) were synthesized in a manner similar to those of Examples 3 and 4.

### <Example 6>

### <Evaluation of calcium receptor activation activity>

For evaluation of calcium receptor activation activity, a Ca ion concentration-dependent Cl ionic current measuring method using a *Xenopus laevis* oocyte expression system was used. If each activator is added to *Xenopus laevis* oocytes expressing the calcium receptor, intracellular Ca ions increase. Then, the Ca ion concentration-dependent Cl channel opens, and the intracellular current value changes as an ionic current. By measuring the change in this intracellular current value, whether the calcium receptor activation activity is present or not can be known.

Specifically, abdomen of *Xenopus laevis* was opened, and an egg batch was taken out and treated with a 1% collagenase solution at 20°C for 2 hours to obtain individual oocytes. Into the oocytes, 50 nl of 1 µg/µl receptor cRNA or 50 nl of sterilized water per one oocyte was introduced by using a micro glass capillary, and the oocytes were cultured at 18°C for 2 or 3 days. For the culture, a solution obtained by adding 2 mM pyruvic acid, 10 U/ml of penicillin, and 10 µg/ml of streptomycin to the solution mentioned in Example 2 was used. After the culture, a test solution was added to the oocytes introduced with cRNA or sterilized water.
Electrophysiological measurement was performed by using an amplifier, Geneclamp500 (Axon), and recording software, AxoScope 9.0 (Axon). The oocytes were voltage-clamped at - 70 mV by the double electrode voltage clamp method, and the intracellular current was measured via the Ca ion concentration-dependent Cl ion channel. The maximum value of the intracellular current was considered as the response current value.

### <Example 7>

### <Evaluation of calcium receptor activation activity of calcium>

The calcium receptor activation activity of calcium was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, calcium was added (2 mM, 5 mM, 10 mM, 20 mM), and Ca ion concentration-dependent Cl response current was measured. The results are shown in Fig. 1. From these results, it was confirmed that cRNA of the calcium receptor introduced into the oocytes were functionally expressed. Furthermore, since the oocytes introduced with water did not respond to even high concentration calcium, it was confirmed that the calcium receptor is not expressed in the oocytes themselves.

### <Example 8>

### <Evaluation of calcium receptor activation activity of L-amino acids>

Calcium receptor activation activity of L-amino acids was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, alanine (10 mM), arginine (10 mM), asparagine (10 mM), aspartic acid (10 mM), cysteine (10 mM), glutamine (10 mM), glutamic acid (10 mM), glycine (10 mM), histidine (10 mM), isoleucine (10 mM), leucine (10mM), lysine (10 mM), methionine (10 mM), phenylalanine (10 mM), proline (10 mM), serine (10 mM), threonine (10 mM), tryptophan (10 mM), tyrosine (10 mM), valine (10 mM), ornithine (10 mM), taurine (10 mM), or hydroxyproline (10 mM) was added, and Ca ion concentration-dependent Cl response current was measured. The results are shown in Fig. 2. By these results, it was demonstrated that cysteine, histidine, phenylalanine, tryptophan, and tyrosine had definite calcium receptor activation activity. As for the aforementioned amino acids, the activation activity was reported in Proc. Natl. Acad. Sci. USA, 2000 Apr. 25, 97(9):4814-9.

### <Example 9>

### <Evaluation of calcium receptor activation activity of D-cysteine>

Calcium receptor activation activity of D-cysteine was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, D-cysteine (10 mM), L-cysteine (10 mM), D-tryptophan (10 mM), or L-tryptophan (10 mM) was added, and Ca ion concentration-dependent Cl response current was measured. The results are shown in Fig. 3. By these results, it was demonstrated that D-cysteine had definite calcium receptor activation activity.

### <Example 10>

### <Evaluation of calcium receptor activation activity of peptides>

Calcium receptor activation activity of peptides was evaluated by using the method described in Example 6. That is, oocytes introduced with cRNA of the calcium receptor or sterilized water were prepared, and voltage-clamped at -70 mV by the double electrode voltage clamp method. To the voltage-clamped oocytes, γ-Glu-Cys-Gly (50 µM), γ-Glu-Cys(SNO)-Gly (50 µM), γ-Glu-Ala (50 µM), γ-Glu-Gly (500 µM), γ-Glu-Cys (50 µM), γ-Glu-Met (500 µM), γ-Glu-Thr (50 µM), γ-Glu-Val (50 µM), γ-Glu-Orn (500 µM), Asp-Gly (1 mM), Cys-Gly (1 mM), Cys-Met (1 mM), Glu-Cys (50 µM), Gly-Cys (500 µM) or Leu-Asp (1 mM) was added, and Ca ion concentration-dependent Cl response current was measured. The results are shown in Fig. 4. By these results, it was demonstrated that the aforementioned peptides had definite calcium receptor activation activity.

### <Example 11>

### <Evaluation of calcium receptor activation activity of peptides>

Calcium receptor activation activity of peptides was evaluated in the same manner as that of Example 10. Each of the peptides shown in Table 2 was added to voltage-clamped oocytes at 1000 µM, 300 µM, 100 µM, 30 µM, 10 µM, 3 µM, 1 µM, 0.3 µM, and 0.1 µM, and Ca ion concentration-dependent Cl response current was measured. The lowest concentration with which current was detected was shown in Table 2 as the activity. From these results, it became clear that these 32 kinds of peptides had calcium receptor activation activity.

**Table 2**

| No. | Peptide | Activity |
|---|---|---|
| 1 | γ-Glu-Met(O) | 1000 *µ*M |
| 2 | γ-Glu-Val-Yal | 1000 *µ*M |
| 3 | γ-Glu-val-Glu | 1000 *µ*M |
| 4 | γ-Glu-Val-Lys | 1000 *µ*M |
| 5 | γ-Glu-Val-Arg | 1000 *µ*M |
| 6 | γ-Glu-Vel-Asp | 1000 *µ*M |
| 7 | γ-Glu-Val-Met | 1000 *µ*M |
| 8 | γ-Glu-Val-Thr | 1000 *µ*M |
| 9 | γ-Glu- γ-Glu-Val | 1000 *µ*M |
| 10 | γ-Glu-Val-NH2 | 1000 *µ*M |
| 11 | γ-Glu-Val-ol | 1000 *µ*M |
| 12 | γ-Glu-Ser | 300 *µ*M |
| 13 | γ-Glu-Tau | 300 *µ*M |
| 14 | γ-Glu-Cys(S-Me)(O) | 300 *µ*M |
| 15 | γ-Glu-Val-His | 100 *µ*M |
| 16 | γ-Glu-Val-Orn | 100 *µ*M |
| 17 | γ-Glu-Leu | 100 *µ*M |
| 18 | γ-Glu-Ile | 100 *µ*M |
| 19 | γ-Glu-t-Leu | 100 *µ*M |
| 20 | γ-Glu-Cys(S-allyl)-Gly | 100 *µ*M |
| 21 | γ-Glu-Val-Asn | 30 *µ*M |
| 22 | γ-Glu-Gly-Gly | 30 *µ*M |
| 23 | γ-Glu-Val-Phe | 30 *µ*M |
| 24 | γ-Glu-Val-Ser | 30 *µ*M |
| 25 | γ-Glu-Val-Pro | 30 *µ*M |
| 26 | γ-Glu-Ser-Gly | 30 *µ*M |
| 27 | γ-Glu-Cys(S-Me) | 30 *µ*M |
| 28 | γ-Glu-Val-Cys | 10 *µ*M |
| 29 | γ-Glu-Val-Gln | 10 *µ*M |
| 30 | γ-Glu-Abu-Gly | 3 *µ*M |
| 31 | γ-Glu-Cys(S-Me)-Gly | 3 *µ*M |
| 32 | γ-Glu-Val-Gly | 0.1 *µ*M |

### <Example 12>

### <Distribution of the calcium receptor in a living body>

In order to elucidate distribution of the calcium receptor in a living body, expression of the calcium receptor in tissues was investigated as follows by using RNAs obtained from rats as a material and the quantitative RT-PCR method.

RNAs were prepared from rat tissues as follows. From 15-week old male F344 rats, cerebrum, cerebellum, lung, heart, liver, kidney, adrenal body, thyroid, parathyroid, pancreas, spleen, esophagus, upper third of stomach, fundus of stomach, duodenum, jejunum, ileum, caecum, colon, rectum, testis, epididymis, bladder, bone marrow, gastrocnemial muscle, soleus, skeletal muscle, fat, prostate, tongue, sublingual gland, and thymus were isolated. Total RNA was prepared by using Isogen (Nippon Gene). Each tissue was homogenized by using Fast Prep (BIO101) or Polytron homogenizer, and total RNA was extracted from the homogenate. By using the total RNA as a template, oligo dT primers and Superscript III reverse transcriptase (Invitrogen), cDNA was synthesized. The primers shown in Table 1 (rCASR_f: SEQ ID NO: 3 and rCASR_r: SEQ ID NO: 4) were synthesized, and RT-PCR of the calcium receptor was performed as follows. By quantitative PCR using cDNA of each tissue as a template, SYBR Green Realtime PCR Master Mix (TOYOBO), and ABI PRISM 7700 Sequence Detector, the expression amount of the calcium receptor gene was analyzed. Distribution of tissue expression is shown in Table 3. Only for pancreas, human total RNA (Stratagene) was used.

From these results, it was confirmed that the calcium receptor was widely expressed in a living body, and not only in the parathyroid and kidney. This suggested that the calcium receptor was involved not only in functions of the parathyroid and kidney but also in various physiological functions of peripheral tissues and peripheral organs.

**Table 3 : Distribution of calcium receptor in living body**

| **CaSR** | |
|---|---|
| Cerebrum | ○ |
| Cerebellum | ○ |
| Lung | × |
| Heart | ○ |
| Liver | ○ |
| Kidney | ○ |
| Adrenal body | × |
| Thyroid | ○ |
| Pancreas | ○ |
| Spleen | ○ |
| Esophagus | ○ |
| Upper of stomach | ○ |
| Fundus of stomach | ○ |
| Duodenum | ○ |
| Jejunum | ○ |
| Ileum | ○ |
| Caecum | ○ |
| Colon | ○ |
| Rectum | ○ |
| Testis | ○ |
| Epididymis | ○ |
| Bladder | × |
| Bone marrow | ○ |
| Gastrocnemial muscle | ○ |
| Soleus | ○ |
| Skeletal muscle | ○ |
| Fat | ○ |
| Prostate | × |
| Tongue | ○ |
| Sublingual gland | ○ |
| Thymus | ○ |
| Aorta | ○ |

### <Example 13>

### <Pharmacological evaluation of calcium receptor activators using animals>

Pharmacological effects of the peptides were confirmed by animal study. Parathyroid hormone (PTH) is one of the major proteins which relate to calcium homeostasis. Increase of PTH-amount is directly responsible for hyperparathyroidism and hypoparathyroidism. It is known that PTH is secreted from the parathyroid gland, and its secretion is inhibited when calcium receptor is activated. Male, 10 weeks old, Sprague-Dawley strain rats were used. "Rat Intact PTH ELISA KIT"(Immutopics) was used for measuring PTH according to the producer's recommended protocol. 100mg/ml of γ-Glu-Val-Gly was dissolved in physiological saline, and 0.1 ml/100 g body weight for each was injected through the tail vein. Blood was collected from the vein under right clavicle, and blood serum was obtained by centrifugation. Each group consists of 3 rats, and resulted PTH variation is shown below as average value±standard error. The result for peptide-administered group was: 198±72.5pg/ml before the administration and 9.44±2.94pg/ml 15 minutes after the administration; meanwhile, the result for control group (physiological saline administered group) was: 89.4±49.5pg/ml before the administration and 41.1±11.6pg/ml 15 minutes after the administration. As a result, decreased PTH level was shown by the administration of the peptides of the present invention.

### Industrial Applicability

Receptor activators for the GPCR (G-protein coupling type receptor) are universally applicable as curative agents of various diseases. Examples of curative commercial agents so far, or those under research, include the acetylcholine receptor activator (for treating Alzheimer disease), the adrenoreceptor activator (for treating bronchial asthma, for treating obesity), the opioid receptor activator (for treating pain diseases), the receptor activator (for treating pain diseases), the calcitonin receptor activator (for treating osteoporosis), the cholecystokinin receptor activator (for treating diabetes), the serotonine receptor activator (for treating migraine), the dopamine receptor activator (for treating Parkinson's disease, sedative), the melatonin receptor activator (for treating primary insomnia), the melanocortin receptor activator (for treating obesity), and so forth.

As for the range of applications of the calcium receptor activator belonging to GPCR as a curative agent, since the calcium receptor is distributed over a wide range of tissues as shown in Example 12, Table 3, it is expected that it is applicable to a wide range.of diseases.

## Claims

1. A composition comprising one or more kinds of substances selected from γ-Glu-Cys, γ-Glu-Cys(SNO)-Gly, γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-Glu-Val-Lys, γ-Glu-γ-Glu-Val, γ-Glu-Val-NH₂, γ-Glu-Val-ol, γ-Glu-Ser, γ-Glu-Tau, γ-Glu-Cys(S-Me)(O), γ-Glu-Leu, γ-Glu-Ile, γ-Glu-t-Leu, γ-Glu-Cys(S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys(S-Me), γ-Glu-Abu-Gly and γ-Glu-Cys(S-Me)-Gly for use in therapeutically reducing blood calcium level or parathyroid hormone level.

2. The composition for use according to claim 1, which further contains an other compound having calcium receptor activation activity which is a cation.

## Patentansprüche

1. Zusammensetzung, welche eine oder mehrere Arten von Substanzen umfasst, die aus γ-Glu-Cys, γ-Glu-Cys(SNO)-Gly, γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met(O), γ-Glu-Val-Val, γ-Glu-Val-Glu, γ-Glu-Val-Lys, γ-Glu-y-Glu-Val, γ-Glu-Val-NH₂, γ-Glu-Val-ol, γ-Glu-Ser, γ-Glu-Tau, γ-Glu-cys(S-Me) (O), γ-Glu-Leu, γ-Glu-Ile, γ-Glu-t-Leu, γ-Glu-Cys(S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys(S-Me), γ-Glu-Abu-Gly und γ-Glu-Cys(S-Me)-Gly ausgewählt sind, zur Verwendung bei der therapeutischen Verringerung des Calciumblutspiegels oder des Parathormonspiegels.

2. Zusammensetzung zur Verwendung nach Anspruch 1, die weiterhin eine andere Verbindung mit Calciumrezeptoraktivierungsaktivität enthält, wobei diese ein Kation ist.

## Revendications

1. Composition comprenant un ou plusieurs types de substances choisies parmi γ-Glu-Cys, γ-Glu-Cys (SNO)-Gly, γ-Glu-Ala, γ-Glu-Gly, γ-Glu-Met, γ-Glu-Thr, γ-Glu-Val, γ-Glu-Orn, Asp-Gly, Cys-Gly, Cys-Met, Glu-Cys, Gly-Cys, Leu-Asp, D-Cys, γ-Glu-Met (O), γ-Glu-Val-Val, γ-Glu-Val-Glu, γ-Glu-Val-Lys, γ-Glu-γ-Glu-Val, γ-Glu-Val-NH₂, γ-Glu-Val-ol, γ-Glu-Ser, γ-Glu-Tau, γ-Glu-Cys (S-Me) (O), γ-Glu-Leu, γ-Glu-Ile, γ-Glu-t-Leu, γ-Glu-Cys (S-allyl)-Gly, γ-Glu-Gly-Gly, γ-Glu-Val-Phe, γ-Glu-Val-Ser, γ-Glu-Val-Pro, γ-Glu-Val-Arg, γ-Glu-Val-Asp, γ-Glu-Val-Met, γ-Glu-Val-Thr, γ-Glu-Val-His, γ-Glu-Val-Asn, γ-Glu-Val-Gln, γ-Glu-Val-Cys, γ-Glu-Val-Orn, γ-Glu-Ser-Gly, γ-Glu-Cys (S-Me), γ-Glu-Abu-Gly et γ-Glu-Cys (S-Me)-Gly destinée à être utilisée dans la réduction thérapeutique de la concentration sanguine de calcium ou de la concentration sanguine d'hormone parathyroïdienne.

2. Composition destinée à être utilisée selon la revendication 1, qui contient en outre un autre composé ayant une activité d'activation des récepteurs de calcium qui est un cation.
